(19) Europäisches Patentamt
European Patent Office
Office européen des brevets

(11) **EP 4 115 795 A1**

(12) **EUROPEAN PATENT APPLICATION**

(43) Date of publication:
**11.01.2023 Bulletin 2023/02**

(21) Application number: **21305948.8**

(22) Date of filing: **08.07.2021**

(51) International Patent Classification (IPC):
**A61B 5/00** (2006.01)  **A61B 5/11** (2006.01)
**A61B 5/16** (2006.01)

(52) Cooperative Patent Classification (CPC):
**A61B 5/6803; A61B 5/0077; A61B 5/1116;**
**A61B 5/16;** A61B 5/112; A61B 5/1121;
A61B 5/4005; A61B 5/4561

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **ESSILOR INTERNATIONAL**
**94220 Charenton-Le-Pont (FR)**

(72) Inventors:
• **SPIEGEL, Daniel**
 **424796 Singapore (SG)**
• **GIRAUDET, Guillaume**
 **45000 ORLEANS (FR)**

(74) Representative: **Jacobacci Coralis Harle**
 **32, rue de l'Arcade**
 **75008 Paris (FR)**

(54) **SYSTEM FOR DETECTING A BODY RESPONSE TO A VISUAL STIMULUS ON AN EYEWEAR AND METHODS FOR THE SYSTEM AND EYEWEAR**

(57)    The disclosure concerns a system and methods for detecting a body response to a visual stimulus. The system includes an eyewear delivering a left visual stimulus via a left side of the eyewear and a right visual stimulus via a right side of the eyewear. The system further includes a sensor configured to measure a body posture of a subject's body when the subject is wearing the eyewear. The system further includes a controlling circuit operably coupleable to the eyewear and to the sensor, so that when operably coupled, the controlling circuit: receives measurements of the body posture from the sensor, provides a body posture response information based on the measurements of the body posture, and determines a deviation of the body posture response information to a reference, where the reference is based on the left visual stimulus and the right visual stimulus.

FIG. 1A

EP 4 115 795 A1

## Description

## TECHNICAL FIELD

Various aspects of this disclosure relate to a system for detecting a body response to a visual stimulus provided by an eyewear. Various aspects of this disclosure further relate to methods for detecting a body response to a visual stimulus provided by an eyewear, and a computer program.

## BACKGROUND

Ocular dominance (also known as interocular sensory imbalance) is an inherent property of the visual system. While small ocular imbalance or dominance does not clinically manifest, large levels of this imbalance may adversely affect binocular function (e.g. stereopsis). In the more extreme cases, such as amblyopia (also known as "lazy eye"), it can also affect monocular visual processing, such as visual acuity and contrast sensitivity, and other visual dependent activities, such as visuomotor tasks.

Currently, ocular dominance is evaluated through subjective methods which provide limited information, have little regard to realistic visual environments and fail to integrate ocular dominance with other sensory modalities. As such, these methods have limited applicability to real world multi-sensory scenarios.

Thus, it is desired to seek better means to objectively evaluate ocular dominance.

## SUMMARY

It is an object of the invention to provide systems and methods to objectively evaluate ocular dominance which are applicable to real world multi-sensory scenarios. In particular, it is an object of the invention to provide systems and methods which evaluate ocular dominance through or with other sensory processes, such as multi-sensory integration during locomotion. The system and methods of the invention may be used for the diagnosis and treatment of the human visual system, for example, to aid or be used in the design of ophthalmic lens(es), in particular for ocular dominance. Also, the system and methods of the invention may be used to evaluate or test the subject's response to such personalized ophthalmic lens(es) to further aid in the diagnosis and/or treatment of ocular dominance.

A first aspect of the disclosure concerns a system for detecting a body response to a visual stimulus. The system includes an eyewear delivering a left visual stimulus via a left side of the eyewear and a right visual stimulus via a right side of the eyewear. The system further includes a sensor configured to measure a body posture of a subject's body when the subject is wearing the eyewear. The system further includes a controlling circuit operably coupleable to the eyewear and to the sensor, so that when operably coupled, the controlling circuit: receives measurements of the body posture from the sensor, provides a body posture response information based on the measurements of the body posture, and determines a deviation of the body posture response information to a reference, where the reference is based on the left visual stimulus and the right visual stimulus.

According to various embodiments, the eyewear may be configured to modify a visual balance between the left visual stimulus via the left side of the eyewear and the right visual stimulus via the right side of the eyewear. The sensor may further measure the body posture of the subject in response to the modification of the visual balance by the eyewear.

According to various embodiments, the body posture may be a static position or a movement.

According to various embodiments, the modification in visual balance may include a translation, or a rotation of at least one of the left visual stimulus and the right visual stimulus. According to various embodiments, the modification in visual balance may include a combination of a translation and a rotation of at least one of the left visual stimulus and the right visual stimulus.

According to various embodiments, the modification in visual balance may include a change in the luminance of at least one of the left visual stimulus and the right visual stimulus.

According to various embodiments, the modification in visual balance may include a change in the contrast of at least one of the left visual stimulus and the right visual stimulus.

According to various embodiments, the modification in visual balance may include a change in the spatial frequency content of at least one of the left visual stimulus and the right visual stimulus.

According to various embodiments, the modification in visual balance may include a change in a slant of at least one of the left visual stimulus and the right visual stimulus.

According to various embodiments, the sensor may be removably disposable in relation to the subject for providing the measurements of the body posture.

According to various embodiments, the measurements of the body posture may include a set of measurements including a first displacement in a first direction, which may be medial-lateral to the subject. The set of measurements may further include a second displacement in a second direction, which may be anterior-posterior to the subject and perpendicular to the first direction.

According to various embodiments, the body posture response information may be based on the first displacement, the second displacement, a ratio between the first displacement and the second displacement, or a combination thereof.

According to various embodiments, the controlling circuit may be further configured to determine a set of adjustment values, and the controlling circuit may modify the visual balance between the left visual stimulus and the right visual stimulus. The set of adjustment values may include values to minimize the deviation between the body posture response information and the reference, or to reach the reference.

According to various embodiments, the eyewear may include a filter, and the eyewear may be coupled to an external display which may display the visual stimulus. The filter may be configured to alter a property of the visual stimulus such that the visual stimulus may be displayed as the left visual stimulus and the right visual stimulus. According to various embodiments, the filter may be further configured to modify the visual balance between the left visual stimulus and the right visual stimulus.

A second aspect of the disclosure concerns a method for detecting a body response to a visual stimulus. The method includes providing, the left visual stimulus via the left side of the eyewear and the right visual stimulus via the right side of the eyewear, providing the sensor for measuring the body posture of the subject's body when the subject is wearing the eyewear, and measuring the body posture with the sensor. The method also includes producing, using the controlling circuit, the body posture response information based on the measurements of the body posture, and further includes determining, using the controlling circuit, the deviation of the body posture response information to the reference.

According to various embodiments, the method may include modifying, using the controlling circuit, the visual balance between the left visual stimulus via the left side of the eyewear and the right visual stimulus via the right side of the eyewear. The method may further include measuring, using the sensor, the body posture of the subject in response to the modified visual balance.

According to various embodiments, the method may include providing, using the controlling circuit, the set of adjustment values based on the deviation of the body posture response information to the reference. Alternatively, and in accordance with various embodiments, the method may include providing, using the controlling circuit, the modification of the visual balance between the left visual stimulus and the right visual stimulus to determine the set of adjustment values. The set of adjustment values may be provided to minimize the deviation between the body posture response information and the reference, or to reach the reference.

A third aspect of the disclosure concerns a method for detecting a body response to a visual stimulus. The method includes providing, a left visual stimulus via a left side of an eyewear and a right visual stimulus via a right side of the eyewear, and providing a sensor for measuring a body posture of a subject's body when the subject is wearing the eyewear. The method also includes modifying, at least one of the left visual stimulus and the right visual stimulus, and measuring the body posture with the sensor. The method further includes producing, by a microprocessor operably coupled to the eyewear and to the sensor, a body posture response information based on the measurements of body posture, and determining, by the microprocessor or by another microprocessor, a deviation of the body posture response information to a reference. The reference is based on the left visual stimulus and the right visual stimulus.

According to various embodiments, the method may further include a computer program comprising instructions to cause a computing system to execute the steps of the method of the third aspect.

## BRIEF DESCRIPTION OF THE DRAWINGS

The invention will be better understood with reference to the detailed description when considered in conjunction with the non-limiting examples and the accompanying drawings, in which:

- FIGS. 1A shows an exemplary schematic illustration of a use condition of a system 100 for detecting a body response to a visual stimulus 116, in accordance with various embodiments;
- FIGS. 1B to 1D show examples of the dichoptic presentation of a visual stimulus 116 and the fused cyclopean percept in balanced and imbalanced binocular systems, in accordance with various embodiments;
- FIG. 2A shows examples of the various types 200 of body posture 140 of the subject in response to the visual stimulus 116, in accordance with various embodiments;
- FIG. 2B shows an exemplary illustration of the measurements of the body posture 140 of the subject in response to the visual stimulus 116, in accordance with various embodiments;
- FIG. 2C shows a part of the exemplary schematic illustration of the system 100, which provides the body posture response information 150, in accordance with various embodiments;
- FIG. 3 shows an exemplary schematic illustration of a use condition of a system 300, in accordance with various embodiments;
- FIGS. 4A to 4F show examples of schematic illustrations of a modification in visual balance 310, in accordance with various embodiments;
- FIG. 5 shows an example of a schematic illustration of a use of a system 500 for detecting the body response to a visual stimulus 116, and the subsequent modification in visual balance 310, in accordance with various embodiments;
- FIG. 6 shows an exemplary schematic illustration of a use condition of a system 600, in accordance with various embodiments;
- FIGS. 7A and 7B show schematic illustrations of methods 700A, 700B for detecting a body response to a visual stimulus 116, by way of example and in accordance with various embodiments; and
- FIG. 8 shows an exemplary schematic illustration of method 800 for detecting a body response to a visual stimulus 116, in accordance with various embodiments.

## DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. Other embodiments may be utilized and structural, and logical changes may be made without departing from the scope of the disclosure. The various embodiments are not necessarily mutually exclusive, as some embodiments can be combined with one or more other embodiments to form new embodiments.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

The disclosure illustratively described herein may suitably be practiced in the absence of any element or elements, limitation or limitations, not specifically disclosed herein. Thus, for example, the terms "comprising", "including," containing", etc. shall be read expansively and without limitation. The word "comprise" or variations such as "comprises" or "comprising" will accordingly be understood to imply the inclusion of a stated integer or groups of integers but not the exclusion of any other integer or group of integers. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the disclosure. Thus, it should be understood that although the present disclosure has been specifically described in exemplary embodiments and optional features, modification and variation of the disclosure embodied herein may be resorted to by those skilled in the art.

In the context of various embodiments, the articles "a", "an" and "the" as used with regard to a feature or element include a reference to one or more of the features or elements. As used herein, the term "and/or" includes any and all combinations of one or more of the associated listed items.

The reference signs included in parenthesis in the claims are for ease of understanding of the disclosure and have no limiting effect on the scope of the claims.

According to various embodiments, the term "visual stimulus" ("visual stimuli"), as used herein, may refer to the perception of electromagnetic rays (e.g. light), that evoke a specific functional reaction in the eye(s) and brain of a subject to result in the neural processing and construction of cognitive images via the visual pathway. For example, the visual stimulus may refer to an image(s), which may have shape, color and/or depth. As another example, the visual stimulus may be static or may be in motion, for example, moving in a coherent manner, or moving in a random manner with variable speed. Without wishing to be bound by theory, visual reception occurs at the retina of the subject, where photoreceptor cells (e.g. cones and rods) detect the visual stimulus and transduces them into neural impulses which are transferred through the optic nerve to the brain to construct cognitive images.

According to various embodiments, the term "body response", as used herein, may refer to a reaction, or reactions, from the body of the subject. For example, the body response may include a reaction from a part of the body, such as the eye, hand, head, upper or lower limbs (e.g. arms or legs), and/or torso (e.g. trunk) of the subject. As another example, the body response may include a reaction from the entire body of the subject. The body response may also include a reflex, which refers to an involuntary and instantaneous action by a part of, or the entire body of the subject. Examples of reflexes may include the myotatic and/or the optokinetic nystagmus reflex. Within the context of the disclosure, the body response is produced as a reaction to, or in response to the visual stimulus, and may be a body posture.

According to various embodiments, the term "eyewear", as used herein, may refer to an optical article configured to be worn by a user on/in relation to the eye, for example, in front of a user's eye. For example, the eyewear may be selected from the group of: spectacle, sunglass, head mounted device, augmented reality device, virtual reality (VR) device. According to various embodiments, the eyewear may be electronically active (i.e. electronically powered), or may be electronically passive (e.g., not electronically powered, or electronic component free). The eyewear may include one or more lens(es) and a frame.

According to various embodiments, the term "lens" ("lenses") may have corrective power (e.g., a multifocal lens, a prescription lens for ametropic conditions) or may not have corrective power (e.g., a plano lens). According to various embodiments, the lens(es) may be clear, tinted (e.g. grey tint, pink tint, blue tint, brown tint etc.), or may be polarized.

It is understood that any reference herein to a "frame" is to a portion of the eyewear which is a non-lens portion, for example an eyeglass as in an eyewear may include a frame and may include lenses attached to the frame, if not otherwise explicitly indicated, the lenses are not part of the frame.

According to various embodiments, the term "sensor", as used herein, may refer to a device which detects, e.g., measures, a physical property of the body response of the subject. According to various other embodiments, the sensor may be an image sensor. For example, an image sensor may be within a camera (e.g. digital camera) configured to capture a stream of still or moving images (e.g. videos), and may therefore detect the body response (e.g. body posture) of the subject in response to the visual stimulus. As a further example, the body response of the subject (e.g. body posture) may be obtained from the output (e.g. video) of the image sensor. For instance, a software may be used to detect motion, and provide measurements of the body posture of the subject in its field of view. The image sensor may be a solid-state device which converts the light waves into electrical signals to form digital images of the target, and may be: a charge-coupled sensor (e.g. CCD), an active-pixel sensor (e.g. CMOS sensor), LiveMOS sensors. According to various other embodiments, the sensor may be a motion sensor, and may be selected from the group of: accelerometer, gyro-sensor, gesture detector, or in combination thereof. For example, accelerometers may be used to measure linear acceleration and tilt angles of the subject, for example, when the subject is moving. As a further example, the accelerometer may detect a change in the entire body, upper or lower limbs (e.g. when the subject is walking), head (e.g. head tilt) or eye position of the subject. Non-limiting examples of accelerometers include: single accelerometers and multi-axis accelerometers. As a further example, the motion sensor may be a gyro-sensor (e.g. gyroscope) which senses angular velocity, for instance, rotational motion and orientation (e.g. slant). Within the context of the disclosure, the sensor is configured to detect the body response of the subject in response to the visual stimulus.

According to various embodiments, a circuit may include analog circuits or components, digital circuits or components, or hybrid circuits or components. Any other kind of implementation of the respective functions which will be described in more detail below may also be understood as a "circuit" in accordance with an alternative embodiment. A digital circuit may be understood as any kind of a logic implementing entity, which may be special purpose circuitry or a processor executing software stored in a memory, firmware, or any combination thereof. Thus, in various embodiments, a "controlling circuit" may be a digital circuit, e.g. a hard-wired logic circuit or a programmable logic circuit such as a programmable processor, e.g. a microprocessor (e.g. a Complex Instruction Set Computer (CISC) processor or a Reduced Instruction Set Computer (RISC) processor). A "controlling circuit" may also include a processor executing software, e.g. any kind of computer program, e.g. a computer program using a virtual machine code such as e.g. Java.

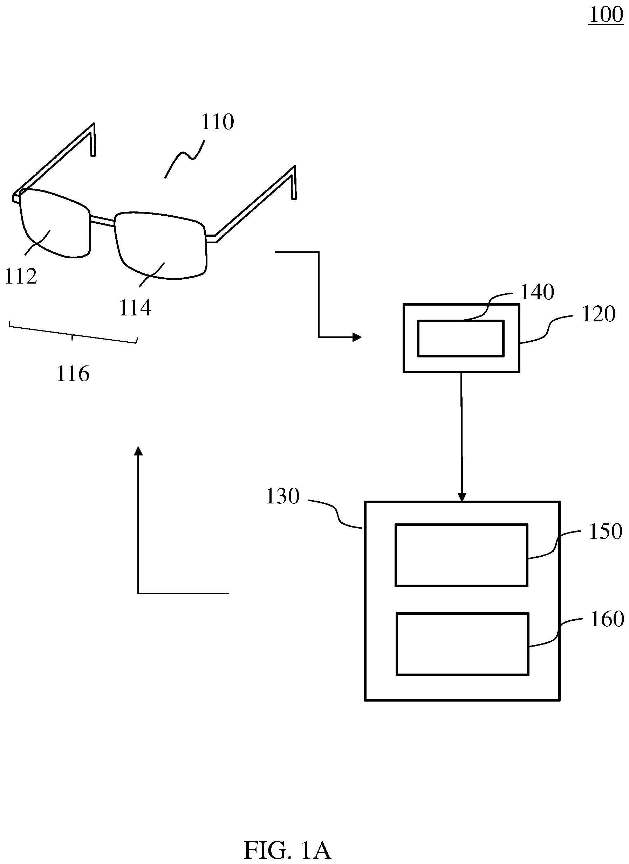

FIGS. 1A to 1D show exemplary schematic illustrations of a use condition of a system 100 for detecting a body response to a visual stimulus 116. FIG. 1A shows an overview of an exemplary system 100. The system 100 includes an eyewear 110, which may be worn by the subject, for example in front of the subject's eye. The eyewear 110 may deliver the visual stimulus 116 to the subject's eyes as a dichoptic visual stimulus, such that the eyewear 110 delivers a left visual 112 via a left side of the eyewear 110, and a right visual stimulus 114 via a right side of the eyewear 110. In other words, the eyewear 110 is configured to dichoptically present the visual stimulus 116, such that the subject views a separate and independent field by each eye. For example, the eyewear 110 delivers the dichoptic presentation of the same visual stimulus 116 (e.g. image) as the left visual stimulus 112 and right visual stimulus 114. As a further example, the left visual stimulus 112 may differ from the right visual 114, for instance, the right visual stimulus 114 may be altered with respect to the orientation (e.g. slant, rotation), position, contrast, luminance, spatial frequency content, motion direction, motion speed, when compared to the left visual stimulus 112, and vice versa.

The system 100 also includes a sensor 120 configured to measure a body posture 140 of a subject's body when the subject wears the eyewear 110, and is thus viewing the left visual stimulus 112 and right visual stimulus 114. The sensor 120, which may include an image or motion sensor, provides measurements of the body posture 140 of the subject in response to the visual stimulus 116. For example, the sensor 120 detects the body posture 140 of the subject and converts the detected measurements into electrical signals, which may be stored in the sensor 120 (e.g. in a memory in the sensor 120) before being transmitted to a controlling circuit 130. In another example, the measurements of the body posture 140 may immediately (e.g. upon acquisition) be transmitted to a controlling circuit 130.

The system 100 further includes a controlling circuit 130 which is operably coupleable to the eyewear 110 and to the sensor 120 such that the controlling circuit 130 when in operation is in communication with the eyewear 110 and the sensor 120. According to various embodiments, the controlling circuit 130 is configured to execute the following steps:

(i) receive measurements of the body posture 140 of the subject, for example, the electrical signals including measurements of body posture 140 provided by the sensor 120; (ii) provide a body posture response information 150 based on the measurements of the body posture 140, for example, by implementing a first calculation algorithm to calculate the body posture response information 150; and (iii) determine, a deviation 160 of the body posture response information 150 to a reference, wherein the reference is based on the left visual stimulus 112 and the right visual stimulus 114, for instance, by implementing a second calculation algorithm to determine the deviation 160.

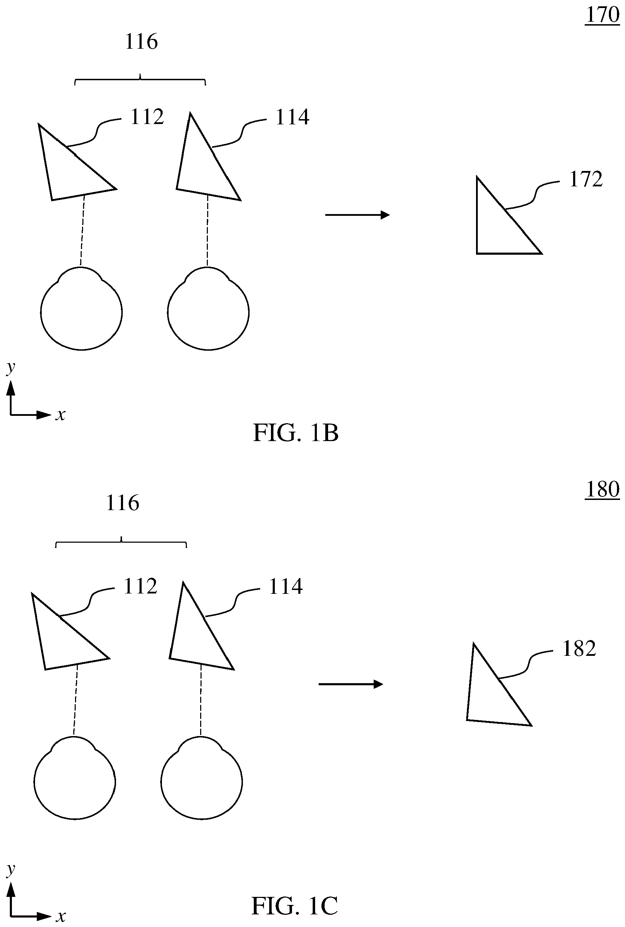

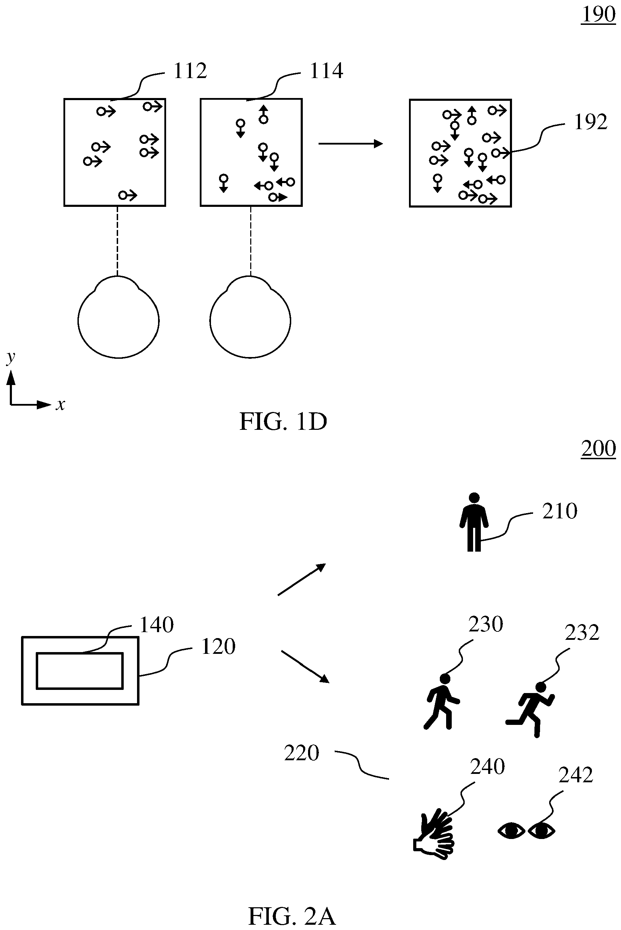

FIGS. 1B to 1D show schematic illustrations of the dichoptic presentation of the visual stimulus 116, by way of example. Referring to FIGS. 1B and 1C, dichoptic presentations 170, 180, of the same static visual stimulus 116 (e.g. image of a shape) may be presented as the left visual stimulus 112 and right visual stimulus 114, and may be rotated (or slanted) in different directions. In another example, a static image may be presented as the left visual stimulus 112, and the right visual stimulus 114 may include the same image but in motion. FIG. 1D shows an exemplary schematic illustration 190 where the left visual stimulus 112 and the right visual stimulus 114 are in motion. For example, the image (e.g. dots) presented as the left visual stimulus 112 may move in a coherent manner (e.g. clockwise or anti-clockwise direction), while the image (e.g. dots) presented as the right visual stimulus 114 may move in a random manner, for instance, in a random dot kinematogram. As a further example, the images presented as the left visual stimulus 112 and the right visual stimulus 114 may move with variable speed, for instance, the dots presented as the left visual stimulus 112 may rotate at 30 °/s, and the dots presented as the right visual stimulus 114 may rotate at 60 °/s. In accordance with various embodiments, non-limiting examples of the visual stimulus 116 may include sinusoidal gratings, kinematograms of various shapes (e.g. dots, squares), or a maze of a movement (e.g. walking) trajectory, path or pattern for a subject. Within the context of the disclosure, the terms "left" and "right" are defined from the subject's reference point, e.g. in relation to the subject.

The integration of ocular dominance and the dichoptic presentation of a visual stimulus will be explained with reference to the examples shown in FIGS. 1B to 1D, which are shown from the reference of a subject. Ocular dominance relates to the tendency to prefer visual input from one eye to the other (e.g. dominant eye of the subject). Currently, ocular dominance may be clinically determined using binocular balance (e.g. ocular weighting), referring to the contribution each eye makes to the binocular percept. Specifically, the human visual system processes and combines the visual stimuli from each eye, for example, the left visual stimulus 112 and the right visual stimulus 114, to form a fused cyclopean percept (e.g. single cognitive image formed by the brain). The fused cyclopean percept varies with the contribution of each eye to the binocular percept and contrasts of the monocular image (also known as contrast-gain control). For example, the orientation or position of the fused cyclopean percept may be the average orientation or position of the two monocular images (e.g. left visual stimulus 112 and right visual stimulus 114) weighted by ocular dominance and their contrasts. Thus, the present disclosure allows for the objective evaluation of ocular dominance, for example, as an amount of the perceptual imbalance. Consequently, ocular dominance may be described objectively, for example, as the amount, quantification, or percentual of imbalance or ocular dominance. In other words, ocular dominance may not only be described in a binary manner, such as being "left or right eye dominant".

According to various embodiments, the term "positive (+) angle θ" may refer to a clockwise rotation or slant from the subject's reference. Similarly, the term "negative (-) angle θ" may refer to an anti-clockwise or counter-clockwise rotation or slant from the subject's reference. For example, a rotation or slant of +30 ° may refer to a clockwise rotation or slant of 30 ° of an image, from the subject's reference. As a further example, a rotation or slant of - 30 ° may refer to an anti-clockwise rotation or slant of 30 ° of an image, from the subject's reference. In connection with angles, the expression "from a[the] subject's reference" may mean that the angle is shown and may be measured from a position of the subject wearing the eyewear while looking at the drawings, it does not mean the subjective cognitive perception of a subject.

FIG. 1B shows the dichoptic presentation 170 of a visual stimulus 116 and the fused cyclopean percept 172 in a balanced binocular system, where each eye makes an equal contribution to the binocular percept. A dichoptic visual stimulus 116 with the same contrast but opposite rotation or slant, for example, the left visual stimulus 112 is rotated or slanted - 10 ° along the x-axis (e.g. a horizontal plane) and a right visual stimulus 114 rotated or slanted +10 ° along the x-axis may be presented via the left eye and right eye, respectively, which are shown from the reference of the subject. In a subject with a balanced binocular system, the fused cyclopean percept 172 is of the image with 0 ° rotation or slant (i.e. zero orientation), since each eye makes an equal contribution to the fused cyclopean percept 172. Conversely, FIG. 1C shows the dichoptic presentation 180 of a visual stimulus 116 and the fused cyclopean percept 182 in an imbalanced binocular system. Similar to FIG. 1B, a visual stimulus 116 with the same contrast is presented in a dichoptic manner with equal but opposite rotations or slants. The fused cyclopean percept 182 would not have a 0 ° rotation or slant (i.e. zero orientation) but an orientation favoring the monocular percept of the dominant eye, which may for example, be the right eye of the subject (e.g. "right eye dominant") in FIG. 1C. For example, the fused cyclopean percept 182 is rotated or slanted +5 ° and thus orientated in favor of the right visual stimulus 114. FIG. 1D shows the dichoptic presentation 190 of a visual stimulus 116 of a random dot kinematogram and the exemplary fused cyclopean percept 192. The left visual stimulus 112 (e.g. dots of a certain contrast) is presented as moving in a coherent direction, while the right visual stimulus 114 (e.g. dots of the same contrast) is presented as moving in random directions. In a subject with a balanced binocular system (e.g. balanced motion coherence threshold), when provided with a certain combination of the left visual stimulus 112 and the right visual stimulus 114 of an image with a certain contrast and numerosity (e.g. number of dots), and based on the fused cyclopean percept 192, the subject may correctly determine the direction in which the coherent dots are moving. Conversely, in a subject with an imbalanced binocular system (e.g. high motion coherence threshold), when provided with the same combination of the left visual stimulus 112 and the right visual stimulus 114 of the image with the certain contrast and numerosity, and based on the fused cyclopean percept 192, the subject may not be able to accurately determine the direction in which the coherent dots are moving. Instead, the fused cyclopean percept 192 may favor the monocular percept of the dominant eye. A manipulation of the contrast and/or the numerosity of either the left visual stimulus 112 and/or the right visual stimulus 114 may allow the subject to correctly determine the direction in which the coherent dots are moving.

Advantageously, the present disclosure provides systems and methods to objectively evaluate ocular dominance by using (i) visual stimuli relevant to real world scenarios, for example, mazes providing a movement trajectory or path; and by (ii) integrating binocular balance (e.g. ocular weighting) with other sensory processes (e.g. body balance, gait, movement of the subject). The present disclosure provides systems and methods to objectively evaluate ocular dominance by measuring body posture 140, providing the body posture response information 150 and determining the deviation 160 of the body posture response information 150 to the reference, wherein the deviation 160 is proportional to the degree of binocular imbalance. Thus, the present disclosure presents systems and methods for evaluating ocular dominance through other sensory processes, for example, by integrating ocular dominance with other sensory modalities, which therefore are more applicable to real world multi-sensory scenarios.

FIG. 2A shows examples of various types 200 of body posture 140 of the subject in response to the visual stimulus 116. According to various embodiments, the body posture 140 is measured by the sensor 120, and may be a static position 210 or a movement 220. According to various embodiments, the static position 210 may include a resting or stationary position (e.g. lack in movement and/or action). For example, the static position 210 may include a halt or sudden stop during a movement 220, for instance, when the subject brakes and comes to an abrupt stop while walking 230, or stops moving his limbs in response to the visual stimulus 116. According to various embodiments, the body posture 140 may also be a movement 220, for example, movement during locomotion. For example, the movement 220, may be the movement 220 of the entire body of the subject, for instance, a walking 230 or running 232 position. In another example, the movement 220 may be the movement 220 of a part of the subject's body, for instance, the movement 220 of the limbs (e.g. arms or legs) or torso. As a further example, the movement 220 of a part of the body may include hand 240 (e.g. grasping or gesture), eye 242, head and/or neck, movements. The movement 220 may also include reflexes.

According to various embodiments, the sensor 120 detects body posture 140 which may include the static position 210 and the movement 220. For example, the sensor 120 may measure the center-of-pressure location of the subject's body posture 140. As a further example, the sensor 120 may measure postural adjustments/responses, such as the subject's gait (e.g. subject's manner during walking 230) and/or body balance (e.g. center of gravity).

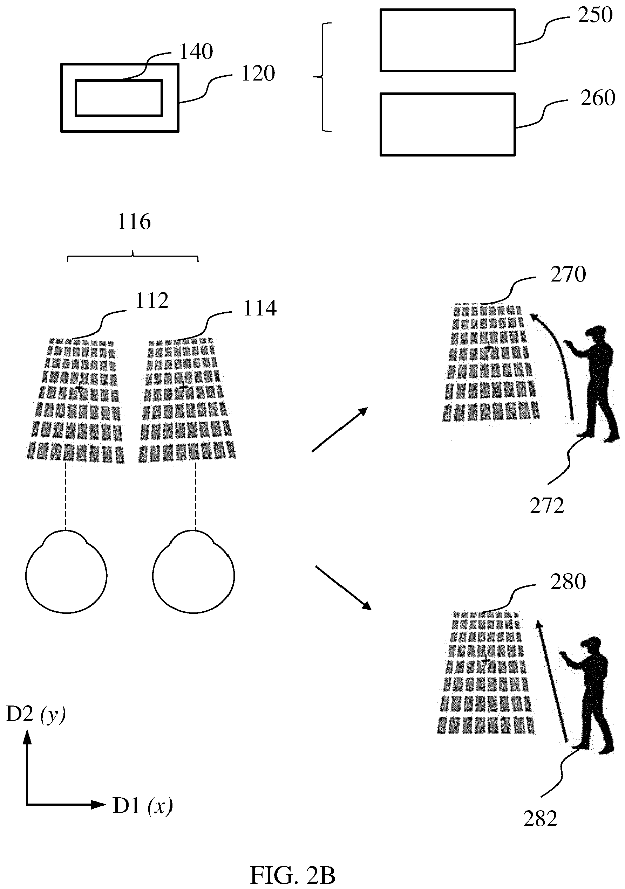

FIG. 2B shows an exemplary illustration of the measurements of the body posture 140 of the subject in response to the visual stimulus 116. According to various embodiments, measures of body posture 140 may include a set of measurements including a first displacement 250 in a first direction D1 which may be medial-lateral (e.g. along the x-axis) to the subject. For example, the first displacement 250 may include the lateral sway or side-to-side movement of the subject during a movement 220. The set of measurements may also include a second displacement 260 in a second direction D2, which may be anterior-posterior (e.g. along the y-axis) to the subject, and may be perpendicular to the first direction D1. For example, the second displacement 260 may include the forward-backward sway or forward-backward movement (e.g. walking 230) of the subject during a movement 220. To illustrate, a subject may be presented with a dichoptic visual stimulus 116 of a maze (e.g. walking trajectory). The left visual stimulus 112 may be slanted -5 ° (e.g. 5 ° anti-clockwise, shown from the reference of the subject), and the right visual stimulus 114 may be slanted +5 ° (e.g. 5 ° clockwise, shown from the reference of the subject), and both the left visual stimulus 112 and right visual stimulus 114 may be of the same contrast. In a subject with an imbalanced binocular system, fused cyclopean percept 270 causes the subject to walk forward and compensate for a perceived slant 272, for example, the subject walks forward and compensates for a perceived +2 ° slant. In other words, the subject's movement 220, which is detected by the sensor 120, may include the first displacement 250 in the first direction D1 (e.g. medial-lateral), and the second displacement 260 in the second direction D2 (e.g. anterior-posterior). Conversely, in a subject with a balanced binocular system, the fused cyclopean percept 280 causes the subject to walk forward without any slant 282, for example, without the first displacement 250 in the first direction D1 (e.g. 0 ° sway). In other words, the set of measurements includes the second displacement 260 in the second direction D2 (e.g. forward, straight motion) but not the first displacement 250.

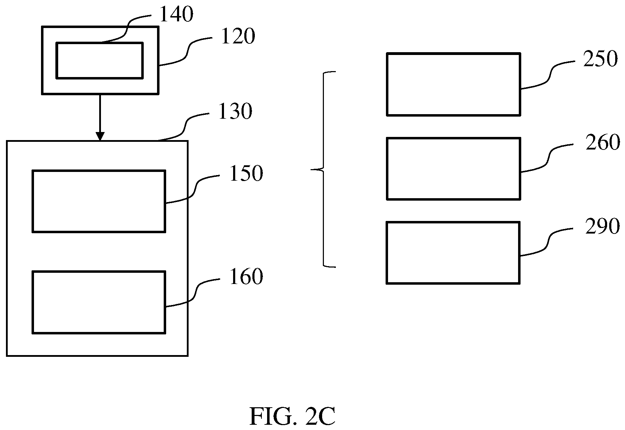

FIG. 2C shows a part of the exemplary schematic illustration of the system 100, which provides the body posture response information 150. The controlling circuit 130 is configured to provide the body posture response information 150 based on the set of measurements of the body posture 140 in response to the visual stimulus 116. According to various embodiments, measurement of the body posture 140 may be transmitted to the controlling circuit 130 in accordance with a pre-defined communication protocol, which may include wireless communications. The sensor 120 and controlling circuit 130 may thus be equipped with the required hardware and/or software protocols to transmit and receive the measurements of the body posture 140. Examples of the pre-defined communication protocol include: Wi-Fi, Bluetooth, ZigBee, SigFox, LPWan, LoRaWan, GPRS, 3G, 4G, LTE, and 5G communication systems. For instance, the sensor 120 may include a wireless communicator configured to transmit the measurements of body posture 140 to the controlling circuit 130 via wireless communications. Alternatively, it may also be envisioned that the sensor 120 may be configured to transmit the measurements of body posture 140 via wired communications (e.g. electrical cables).

According to various embodiments, the body posture response information 150 may be based on the first displacement 250 in the first direction D1, which is medial-lateral to the subject's body. For example, the body posture response information 150 may be calculated according to the first calculation algorithm using the first displacement 250 of the lateral side-to-side movement of the subject. According to another embodiment, the body posture response information 150 may be based on the second displacement 260 in the second direction D2, which is anterior-posterior to the subject's body. For example, the body posture response information 150 may be calculated using the second displacement 250 of the forward or backward movement of the subject. According to another embodiment, the body posture response information 150 may be based on the ratio 290 between the first displacement 250 in the first direction D1 and the second displacement 260 in the second direction D2, for example,

$$ratio\ 290 = \frac{first\ displacement\ 250}{second\ displacement\ 260}$$ or the inverse, $ratio\ 290 = \frac{second\ displacement\ 260}{first\ displacement\ 250}$. According to another embodiment, the body posture response information 150 may be a combination of the first displacement 250, the second displacement 260, and the ratio 290 between the first displacement 250 and the second displacement 260. For example, the combination may be the sum of the first displacement 250 and the second displacement 260.

According to various embodiments, the first calculation algorithm may refer to the algorithm implemented by the controlling circuit 130 to determine the body posture response information 150 based on a set of measurements of the body posture 140. For example, the body posture response information 150 may be calculated based on the average (e.g. mean) or median measurements of body posture 140 of the first displacement 250, and/or the second displacement 260.

According to various embodiments, the controlling circuit 130 is further configured to determine the deviation 160 (e.g. deflection or diversion) of the body posture response information 150 to a reference. For example, the controlling circuit 130 may implement a second calculation algorithm, which may by a subtraction of the body posture response information 150 to the reference, to determine the deviation 160. The deviation 160 may be proportional to the degree of binocular imbalance and ocular dominance of the subject. According to various other embodiments, the deviation 160 of the body posture response information 150 to a reference may be determined in another microprocessor (separate to the controlling circuit 130), at a location outside the system 100, for instance, at a control center or in a cloud. For example, the deviation 160 may be calculated in accordance with the second calculation algorithm in another microprocessor (e.g. computer) or the server of a cloud network). In such embodiments, the controlling circuit 130 may be configured to transmit the body posture response information 150 to another microprocessor via wireless or wired communications, and vice versa.

According to various embodiments, the reference is based on the left visual stimulus 112 and the right visual stimulus 114, and may be pre-determined. For example, the left visual stimulus 112 and the right visual stimulus 114 may be presented to induce an optical imbalance in the subject's body posture 140 (e.g. induce postural adjustment) and the reference may be based on said left 112 and right 114 visual stimuli. As a further example, the reference may be pre-determined based on the differences (e.g. degree of rotation, degree of slant, contrast, luminance, spatial frequency content variations) between the left 112 and right 114 visual stimuli. According to various embodiments, the reference may be based on the body posture response information 150 of a balanced binocular system in response to a given visual stimulus 116. For example, the visual stimulus 116 of the same image with identical contrast, and slanted equally in opposite directions (e.g. the left visual stimulus 112 may be oriented -5 ° and the right visual stimulus 114 may be oriented +5 °) may be presented. The body posture response information 150 in a balanced binocular system may thus include measurements of the body posture 140 without or with minimal first displacement 250 (e.g. medial-lateral) when the subject is moving forward. In other words, the reference may be based on the fused cyclopean percept of a subject with a balanced binocular system where each eye makes an equal contribution. Thus, the average orientation of the two monocular images is 0 °, and the slant is perceptually flattened in the fused cyclopean percept.

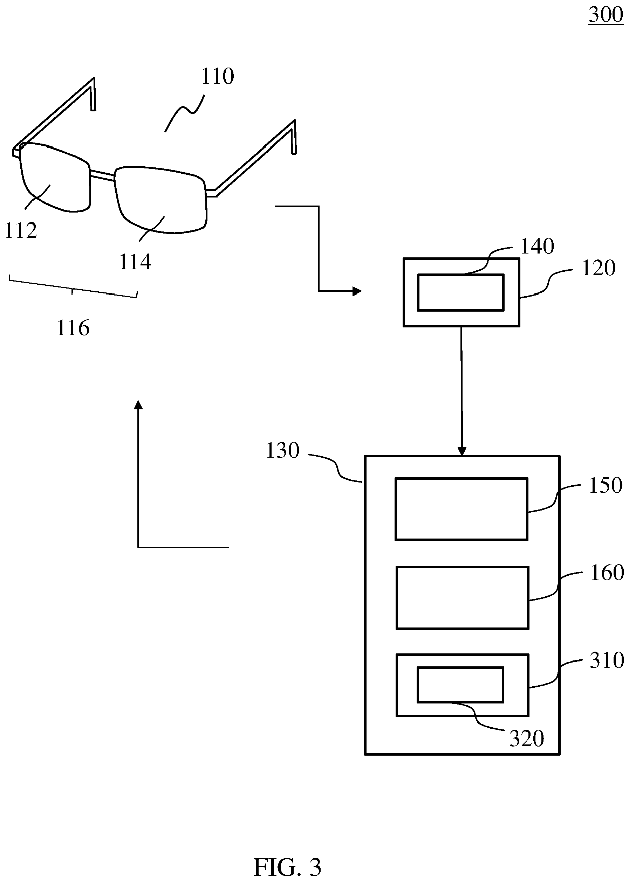

FIG. 3 shows an exemplary schematic illustration of a use condition of a system 300, in accordance with various embodiments. The system 300 may be based on the system 100 as described in relation to FIGS. 1A to 2C, and repeated descriptions will be omitted. The eyewear 110 may be further configured to modify a visual balance 310 between the left visual stimulus 112 via the left side of the eyewear 110 and the right visual stimulus 114 via the right side of the eyewear 110. The modification in visual balance 310 may be delivered to the eyewear 110 to modify the body posture 140 of the subject. For example, as shown in FIG. 3, the controlling circuit 130 may include a module for modifying the visual balance 310 between the left 112 and right 114 visual stimuli. In another example, the modified visual balance 310 may be provided via a filter 610 (e.g. electronically active filter) as will be explained below. The modified visual balance 310 may be configured to induce postural adjustment in the first direction D1 and/or the second direction D2. For example, the modified visual balance 310 between the left visual stimulus 112 and the right visual stimulus 114 may reduce or increase the sensory visual imbalance (e.g. optical imbalance) of the subject. As a further example, the modification of visual balance 310 may produce a determined imbalance in the body posture 140 of the subject.

According to various embodiments, the sensor 120 may further measure the body posture 140 in response to the modification of the visual balance 310 by the eyewear 110. For example, the sensor 120 may further detect the first displacement 250 in the first direction D1 and the second displacement 260 in the second direction D2 of the subject's body posture 140, in response to the modification in visual balance 310.

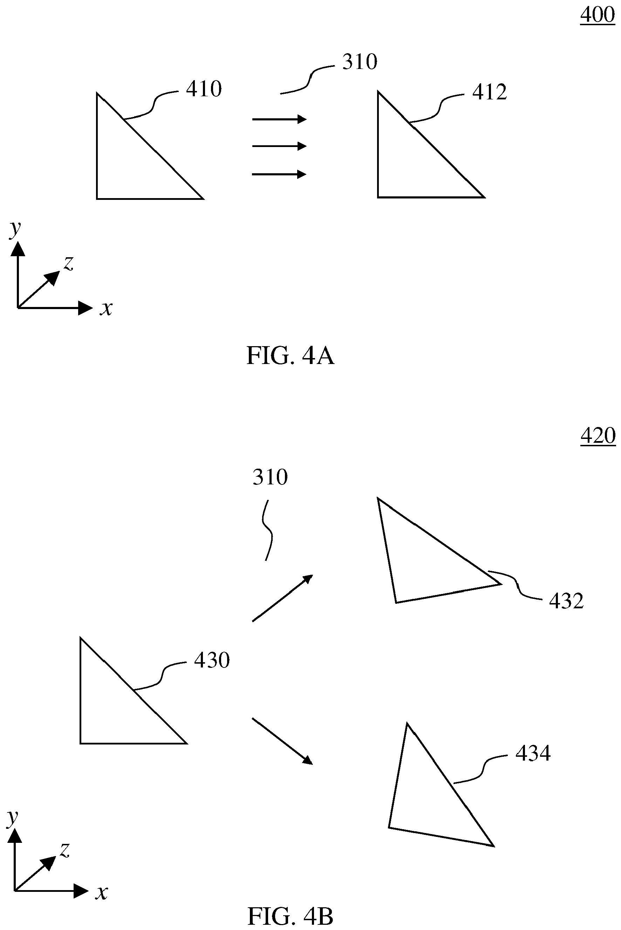

FIGS. 4A to 4F show examples of schematic illustrations of a modification in visual balance 310, in accordance with the various embodiments. In the description below, the modification of the exemplary images is with reference to the x-axis (e.g. horizontal plane), y-axis (e.g. vertical plane) and the z-axis (e.g. perpendicular to x- and y- axes) of the cartesian coordinate system.

According to various embodiments, the modification in visual balance 310 may include a translation 400, of at least one of the left visual stimulus 112 and the right visual stimulus 114. The term, "translation", as used herein, refers to the geometric translation in Euclidean geometry, for example, where every point of the image presented in at least one of the left visual stimulus 112 and the right visual stimulus 114 moves by a same distance in a given direction. Thus, the distances and directions between a set of points of the image is preserved. For example, a translation refers to the shifting of the origin of the coordinate system. In other words, the position of the image may be changed, but the orientation and shape of the image may remain the same. In the example shown in FIG. 4A, the original image 410 shifts in a direction parallel to the x-axis or the horizontal plane (e.g. translation 400 = $f(\Delta x)$). Thus, the modified image 412 may be the same as the original image 410 (e.g. with respect to shape, orientation) with a positional change along the x-axis. In other words, modified image 412 is an isometry (e.g. mapped in different metric position) of the original image 410. Although not illustrated in FIG. 4A, it is further envisioned that the translation 400 may include a vertical translation 400 in the y-axis (e.g. trans-

lation 400 = $f(\Delta y)$), a translation 400 in the z-axis (e.g. translation 400 = $f(\Delta z)$), and may further include a translation 400 with both x-, y- and/or z- coordinates (e.g. translation 400 = $f(\Delta x, \Delta y)$ or $f(\Delta x, \Delta y, \Delta z)$).

According to various embodiments, the modification in visual balance 310 may include a rotation 420, of at least one of the left visual stimulus 112 and the right 114 visual stimulus. The term, "rotation", as used herein, refers to the Euclidean geometric motion of an image about a fixed point (e.g. center or plane of rotation, or the x-, y- and z- axes). For example, an image may be rotated by Euler angles θ in a clockwise or anti-clockwise direction along an axis, which may be from the reference of the subject. Thus, the orientation of the image may be changed, but the shape of the image may remain the same. In the example shown in FIG. 4B, the original image 430 may be rotated -10 ° (e.g. θ = 10 ° in the anti-clockwise direction, shown from the reference of a subject) along the vertical plane (e.g. y-axis) to result in modified image 432. In another example as shown in FIG. 4B, the original image 430 may be rotated +10 ° (e.g. θ = 10 ° in the clockwise direction, shown from the reference of a subject) along the vertical plane (e.g. y-axis) to result in modified image 434. Thus, the shape of the modified images 432, 434 may be the same as the original image 430, and may be rotated by a given angle θ along a fixed point. Therefore, modified images 432, 434 are isometric images of the original image 430. As will be known to those skilled in the art, the rotation of the image may be two-dimensional 2D or three-dimensional 3D using appropriate rotation matrices.

According to various embodiments, the modification in visual balance 310 may include a translation 400 and further include a rotation 420 of the image presented in at least one of the left visual stimulus 112 and the right visual stimulus 114. In accordance with various embodiments, the translation 400 and/or rotation 420 of the image may induce postural adjustments in the body posture 140 of the subject.

According to various embodiments, the eyewear 110 may include physical means to translate 400 and/or to rotate 420 an image. For example, the eyewear 110 may include phase shifters, such as polarizer, which may be placed on the lens(es) of the eyewear 110 to modify the visual balance 310. Alternatively, the translation 400 and/or rotation 420 of at least one of the left visual stimulus 112 and right visual stimulus 114 may be produced by the controlling circuit 130.

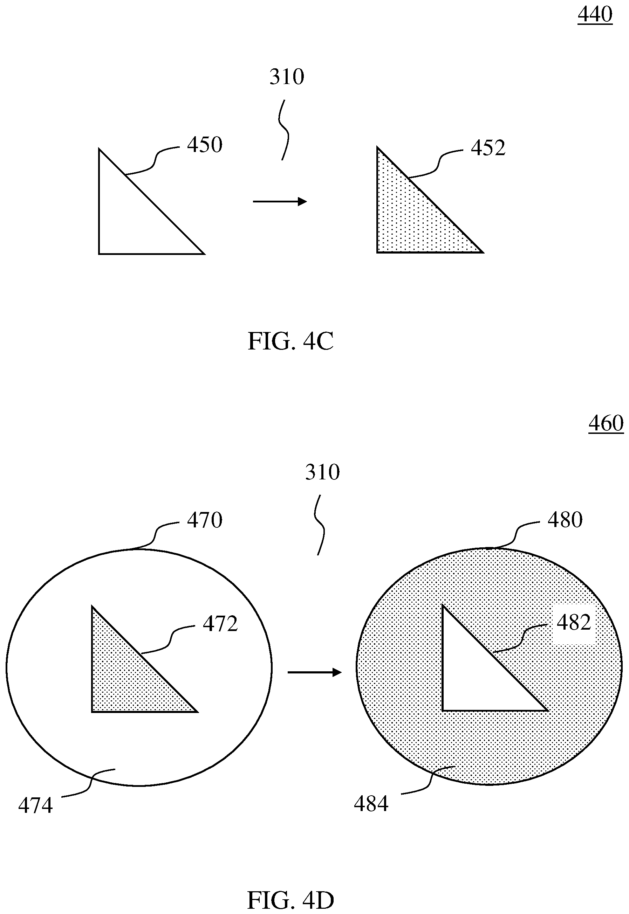

According to various embodiments, the modification in visual balance 310 may include a change in luminance 440 of at least one of the left visual stimulus 112 and the right visual stimulus 114. The term, "luminance", as used herein, may refer to the intensity of light emitted or reflected from the surface of the image per unit area in a given direction, and may be quantified in lux. Referring to FIG. 4C, the luminance of the original image 450 may be modified, for example, may be decreased or increased to produce the modified image 452.

As a further example, the lux of the original image 450 may be decreased or increased to produce the modified image 452, and thus induce postural adjustments in the body posture 140 of the subject.

According to various embodiments, the modification in visual balance 310 may include a change in contrast 460 of at least one of the left visual stimulus 112 and the right visual stimulus 114. The term, "contrast", as used herein, refers to the difference in the color (e.g. greyscale differentiation) and brightness of an object in relation to other objects in the same field of view of a given image, that makes an object (in the image) distinguishable. For example, images having a higher contrast level generally display a greater degree of color or grayscale variation than those of with a lower contrast level. As shown in FIG. 4D, the field of view of the original image 470 may include a dark object 472 on a light background 474. The modification in visual balance 310 may change the contrast 460 of the image to produce a modified image 480 including a light object 482 on a dark background 484. In other words, the contrast between the object(s) and background within the field of view of the image may be changed to produce the modification in visual balance 310. For example, the contrast of at least one of the left visual stimulus 112 and the right visual stimulus 114 may be increased or decreased. As mentioned above, the fused cyclopean percept is a function of the contribution from the two monocular images weighted by ocular dominance and their contrast. Thus, changing the contrast of at least one of the left visual stimulus 112 and right visual stimulus 114 may induce significant postural adjustments to the body posture 140 of the subject.

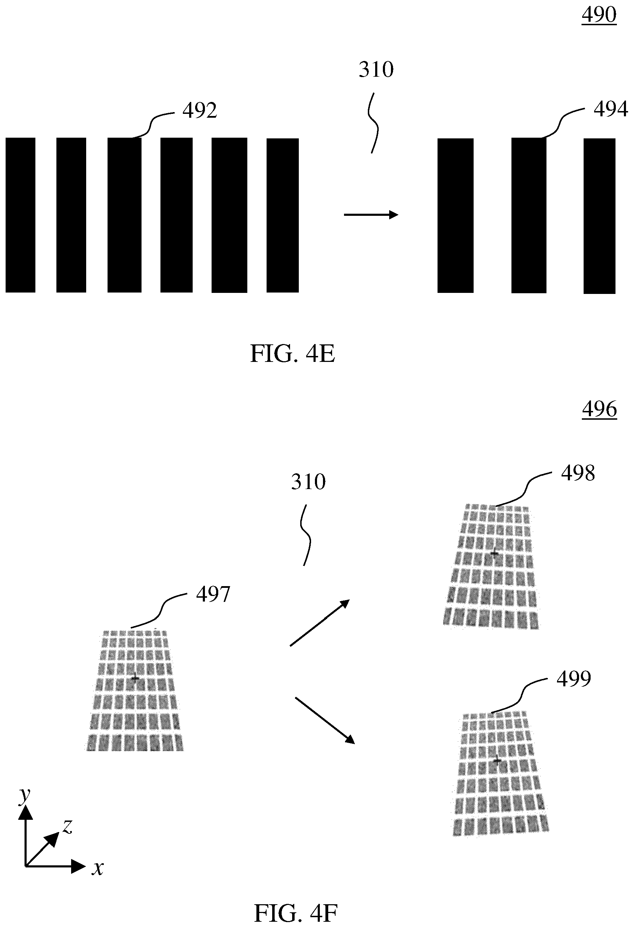

According to various embodiments, the modification in visual balance 310 may include a change in spatial frequency content 490 of at least one of the left visual stimulus 112 and the right visual stimulus 114. The term, "spatial frequency content", as used herein, may refer to the periodic distributions of light and dark within a field of view of a given image. High spatial frequencies may correspond to features such as sharp edges and fine details, whereas low spatial frequencies may correspond to features such as global shape. As shown in FIG. 4E, the original image 492 may be of high spatial frequency, and the modification in visual balance 310 may result in the reduction or lowering of the spatial frequency content in the modified image 494. For example, the modified image 494 may include an image with a reduced or decreased cycle per m (e.g. cycles / m), as compared to the original image 492. In accordance with various embodiments, the change in spatial frequency content 490 may induce postural adjustments in the body posture 140 of the subject.

According to various embodiments, the modification in visual balance 310 may include a slant 496, of at least one of the left visual stimulus 112 and the right 114 visual stimulus. The term, "slant", as used herein, refers to a tilt, slope or incline of an image in a particular direction. For example, an image may be slanted by an angle θ, for instance, an oblique or an acute angle θ in a clockwise or anti-clockwise direction along an axis, which may be from the reference of the subject. Accordingly, the orientation of the image may be changed, but the shape of the image may remain the same. In the example shown in FIG. 4F, the original image 497 may be a maze, for instance, a walking path or trajectory. The original image 497 may be slanted -5 ° (e.g. θ = 5 ° in the anti-clockwise direction, shown from the reference of a subject) to result in modified image 498. In another example as shown in FIG. 4F, the original image 497 may be slanted +5 ° (e.g. θ = 5° in the clockwise direction, shown from the reference of a subject) to result in modified image 499. Thus, the shape (and other characteristics thereof) of the modified images 498, 499 may be the same as the original image 497, but may be slanted by a given angle θ.

According to various embodiments, the eyewear 110 may include physical means to effect changes in the luminance 440, contrast 460, spatial frequency content 490 and/or slant 496 to produce the modification in visual balance 310. For example, the eyewear 110 may include a light intensity pattern adjustment configured to adjust (e.g. decrease or increase) the luminance 440, contrast 460, spatial frequency content 490, and/or slant 496 of an original image. Alternatively, changes in the luminance 440, contrast 460, spatial frequency content 490 and/or slant 496 of at least one of the left visual stimulus 112 and right visual stimulus 114 may be produced by the controlling circuit 130.

According to various embodiments, the modification in visual balance 310 may include at least one of a translation 400, rotation 420, change in luminance 440, change in contrast 460, change in spatial frequency content 490, change in slant 496, or in combination thereof. For example, the modification in visual balance 310 may include a rotation 420 and a change in contrast 460. In another example, the modification in visual balance 310 may include a translation 400 and a change in spatial frequency content 490. In another example, the modification in visual balance 310 may include a slant 496, a change in contrast 460 and a change in spatial frequency content 490. According to various embodiments, the modification in visual balance 310 may include at least one of the left visual stimulus 112 and the right visual stimulus 114. For example, the left visual stimulus 112 may be modified (e.g. change in contrast 460, rotation 420, slant 496) and the right visual stimulus 114 may remain unchanged, and vice versa. As a further example, both the left visual stimulus 112 and the right visual stimulus 114 may be modified, for instance, the left visual stimulus 112 may include a different change to that of the right visual stimulus 114. Alternatively, both the left visual stimulus 112 and the right visual stimulus 114 may be modified in an analogous manner.

Referring to FIGS. 3 to 4F, the controlling circuit 130 may include a module to determine a set of adjustment values 320, which may be provided to modify the visual balance 310 between the left visual stimulus 112

and the right visual stimulus 114. For example, the set of adjustment values 320 may include the values required to produce a translation 400, rotation 420, a change in luminance 440, contrast 460, spatial frequency content 490, slant 496, or combinations thereof, to provide the modification in visual balance 310. In other words, modifying the visual balance 310 of at least one of the left visual stimulus 112 and the right visual stimulus 114 is based on the set of adjustment values 320 determined by the controlling circuit 130.

According to various embodiments, the set of adjustment values 320 may be determined to minimize the deviation 160 between the body posture response information 150 and the reference, or to reach the reference.

In accordance with one embodiment of the present disclosure, the set of adjustment values 320 may be based on the deviation 160 determined by the system 100 as described with reference to FIGS. 1A to 2C. For example, the set of adjustment values 320 may include values equal to the deviation 160, such that when viewing the visual stimulus 116 modified by said adjustment values 320, the body posture 140 and body posture response information 150 of the subject may equal to that of the reference. For instance, the deviation 160 may be determined according to the second calculation algorithm (e.g. subtraction between the body posture response information 150 and the reference).

In accordance with another embodiment, the set of adjustment values 320 may be determined at random to produce the modification in visual balance 310. For example, the controlling circuit 130 may perform a third calculation algorithm to randomly modify the visual balance 310 between the left visual stimulus 112 and the right visual stimulus 114. For example, the controlling circuit 130 may increase or decrease the translation 400, rotation 420, luminance 440, contrast 460, spatial frequency content 490, and/or slant 496 by a pre-determined value to produce the modification in visual balance 310. As a further example, the controlling circuit 130 may provide a 10 % increase or decrease, in a stepwise manner, in the contrast 460, luminance 440, and/or slant 496 of at least one of the left visual stimulus 112 and the right visual stimulus 114. The set of adjustment values 320 may be determined when the body posture 140 of the subject in response to the modification in visual balance 310 reaches that of the reference, or when the body posture 140 of the subject in response to the modification in visual balance 310 is minimized to a pre-determined level (e.g. a level which may be "clinically acceptable", or a level which does not significantly affect the postural balance of the subject).

Therefore, the set of adjustment values 320 may provide the values required to correct or reduce ocular dominance or binocular imbalance in a subject, such that the fused cyclopean percept of the subject may reach or may be that of a balanced binocular system (e.g. equal contribution from both eyes).

Advantageously, the system and methods of the present invention (e.g. via the set of adjustment values 320) may be used to personalize and customize lens(es), and/or to provide personalized treatment for a subject with an imbalanced binocular system. In particular, for the diagnosis and/or treatment of ocular dominance of the human visual system. Further, the present invention may provide the system and methods thereof (e.g. via the set of adjustment values 320) to evaluate or test such personalized or customized lens(es). For example, the system allows for the objective evaluation of said customized lens(es) based on lab-controlled dichoptic input (e.g. visual stimulus 116) and the measured body response of the subject, which may be compared to a reference.

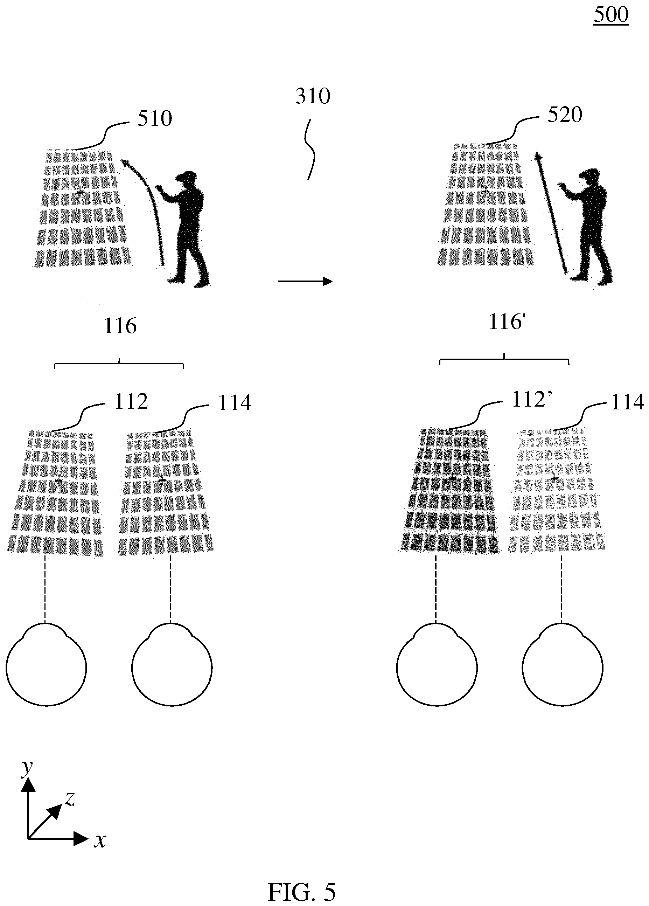

FIG. 5 shows a schematic illustration of a use of a system 500 for detecting the body response to a visual stimulus 116, and the subsequent modification in visual balance 310, by way of example. In system 500, the exemplary subject may be "right-eye dominant", i.e. the monocular percept from the subject's right eye is stronger. The visual stimulus 116, 116' may be a maze of a walking path or trajectory, e.g. flat floor, which is presented as the left visual stimulus 112 and the right visual stimulus 114. Referring to the right side of system 500, the left 112 and right 114 visual stimuli may be identical but slanted in opposite directions. For example, the left visual stimulus 112 may be slanted -5 ° (e.g. 5 ° in the anti-clockwise direction, shown from the reference of a subject) and the right visual stimulus 112 may be slanted +5 ° (e.g. 5 ° in the clockwise direction, shown from the reference of a subject). The fused cyclopean percept 510 of the subject may favor the monocular percept from the stronger eye (e.g. right eye) and the subject may walk in the direction favoring the monocular percept from the stronger eye, for example, with a +2 ° slant. The sensor 120 detects measurements of the subject's body posture 140 which may include the first displacement 250 and the second displacement 260, and provides the measurements of body posture 140 to the controlling circuit 130 which determines the body posture response information 150, and the deviation of the body posture response information 150 to the reference. In the example of FIG. 5, the reference may be the fused cyclopean percept of a balanced binocular system (e.g. without the first displacement 250).

The controlling circuit 130 may further determine the set of adjustment values 320 based on the deviation 160 in accordance with the second calculation algorithm, or may determine the set of adjustment values 320 in accordance with the third calculation algorithm, to modify the visual balance 310 between the left 112 and right 114 visual stimuli. According to various embodiments, the modification in visual balance 310 may be provided to minimize the deviation 160 between the body posture response information 150 and the reference, or to reach the reference. For example, the modification in visual balance 310 may provide a visual stimulus 116' which favors the weaker eye (e.g. left eye), for instance by strengthening the signal (e.g. increasing the contrast and thus providing a contrast offset) of the left visual stimulus 112'. The right visual stimulus 114 may remain unchanged or may be changed as well. In another example (which may also be combined with the previous example), the modification in visual balance 310 may provide a visual stimulus 116' directed to the dominant eye (e.g. right eye), for instance, by weakening the signal (e.g. decreasing the contrast and thus providing a contrast offset) of the right visual stimulus 114. The left visual stimulus 112 may remain unchanged or may be changed as well. In yet another example, the modification in visual balance 310 may include providing a visual stimulus 116" which increases the signal directed to the weaker eye, and/or decreases the signal directed to the dominant eye. For instance, the visual stimulus 116" may include a left visual stimulus 112 with increased contrast and degree of slant, and/or a right visual stimulus 114 with decreased contrast and degree of slant. Thus, the fused cyclopean percept 520 perceived by the subject with the imbalanced binocular system may be similar to that of a balanced binocular system. For example, the body posture 140 of the subject may be equal to the reference (e.g. without measurements of the first displacement 250). In other words, the modification in visual balance 310 provides compensation to the binocularly weaker eye, or perceptually reduces the relative contribution provided by the binocularly dominant eye, such that the fused cyclopean percept 520 is equal to that of a binocularly balanced system.

According to various embodiments, the sensor 120 may be removably disposable in relation to the subject for providing the measurements of the body posture 140. For example, the sensor 120 may be attached to the entire body of the subject, and may include wearable full-body image or motion tracking suits or devices. Accordingly, the sensor 120 provides measurements of the subject's entire body. As a further example, the sensor 120 may be attached to a part of the body of the subject, and measurements of the body posture 140 may be of that particular part of the body. Non-limiting placements of the sensor 120 on a part of the subject's body may include the torso, limbs (e.g. arms or legs), hands, feet, head, neck, eye. As a further example, the sensor 120 may be located on the eyewear 110. In another example, the sensor may be integrated on the eyewear 110. For instance, the eyewear may be a VR device, and the sensor 120 may be integrated on the device and may provide measurements of the body posture 140 of the subject's head or eye(s) in response to the visual stimulus 116. According to various embodiments, the sensor 120 may be located in an external display 620, such as hand-held devices which include game consoles and/or mobile devices, where the perceived binocular imbalance may be provided by measurements of the body posture 140 (e.g. tilt angle of hand when tilting the external display 620). According to various embodiments, the degree of binocular imbalance in response to the visual stimulus 116 may be proportional to the deviation 160 determined by the controlling circuit 130 or another microprocessor, and may be a function of the tilt angle or degree of slant of the subject's body posture 140 which is measured by the sensor 120.

According to various embodiments, the controlling circuit 130 may be located on the eyewear 110. For example, the controlling circuit 130 may be integrated on the frame of the eyewear 100, for instance, the front portion of the frame of the eyewear 110. According to various embodiments, the controlling circuit 130 may be located with the sensor 120, for example, the sensor 120 and controlling circuit 130 may be integrated into the same circuit. According to various embodiments, the controlling circuit 130 may be external to the eyewear 110 and/or the sensor 120, for example, as a separate device within system 100, 300, 500. For example, the controlling circuit 130 may be in a separate microprocessor operably coupled to the eyewear 110 and sensor 120. Alternatively, the controlling circuit 130 may be located outside the system 100, 300, 500, for instance, in a control center (e.g. at another location) or in a server in a cloud network. The controlling circuit 130 may receive measurements of body posture 140 via wired or wireless communications, in accordance with various embodiments.

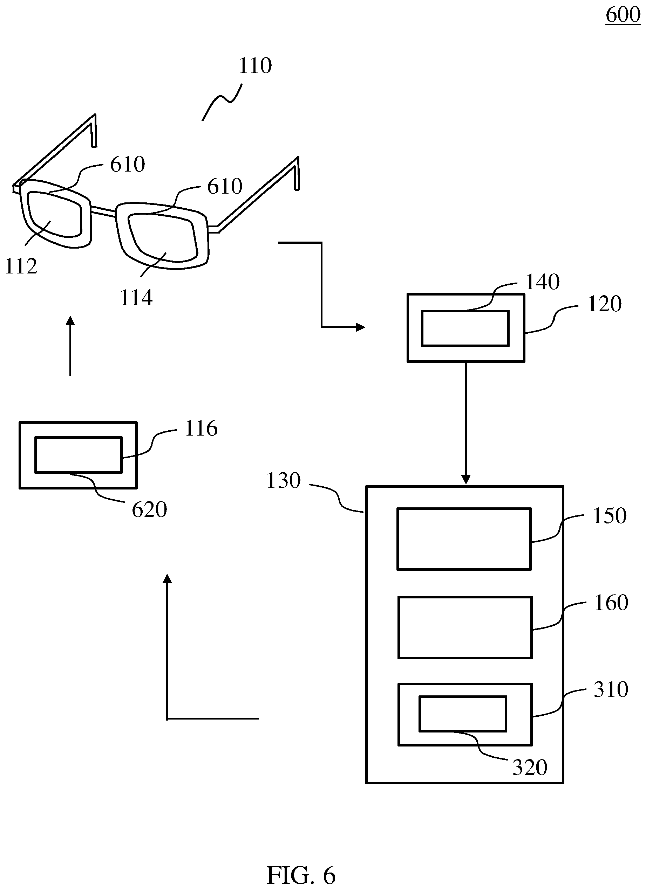

FIG. 6 shows an exemplary schematic illustration of a use condition of a system 600, according to various embodiments. The system 600 may be based on the system 100, 300 described in relation to FIGS. 1A to 4F, and repeated descriptions will be omitted for brevity. The system 600 may include an external display 620 configured to display the visual stimulus 116. According to various embodiments, the external display 620 may be an electronic device including a visual display unit (e.g. monitor, screen) to display the visual stimulus 116. The external display 620 may be portable and may include hand-held devices such as smartphones, laptops, tablets, or game consoles. Alternatively, the external display 620 may be attached at a fixed position, and may include projector screens. According to various embodiments, the controlling circuit 130 may control the visual stimulus 116 presented on the external display 620. According to various other embodiments, the visual stimulus 116 presented on the external display 620 may be controlled via an external processor (e.g. computer, server of a cloud network via wireless communications), or controlled by a processor within the external display 620 (e.g. microprocessor of the hand-held device).

The eyewear 110 may further include a filter 610, and the eyewear 110 may be coupled to the external display 620. For example, the eyewear 110 may allow the subject to view the visual stimulus 116 displayed on the external display 620. The filter 610 (e.g. optical filter) may be configured to alter a property of the visual stimulus 116 such that the visual stimulus 116 is displayed as the left visual stimulus 112 and the right visual stimulus 114. In other words, the filter 610 may display the visual stimulus 116 as a dichoptic presentation. According to various embodiments, the filter 610 may be electronically passive or active. Passive filters 610 may include viewing aids which use prisms, mirrors or lenses to alter the property of the visual stimulus 116. Examples of passive filters 610 include polarization system configured to only allow light waves of a specific polarization to pass through (e.g. polarizer), lenses including microscopically small prisms (e.g. prismatic foils), and anaglyphic presentation (e.g. anaglyph 3D goggles). Alternatively, active filters 610 may include filters which may be controlled by the controlling circuit 130, or an external processor (e.g. computer, server of a cloud network), to alter the property of the visual stimulus 116 such that it is displayed as the left visual stimulus 112 and the right visual stimulus 114.

According to various embodiments, the filter 610 may further be configured to modify the visual balance 310 between the left visual stimulus 112 and the right visual stimulus 114. For example, the controlling circuit 130 may modify the visual balance 310 based on the set of adjustment values 320, and may provide instructions such that the active filter 610 modifies the luminance 440, contrast 460, spatial frequency content 490, slant 496, and/or translates 400, rotates 420 at least one of the left visual stimulus 112 and the right visual stimulus 114. As a further example, a passive filter 610 may be physically altered to provide the modification in visual balance 310. For instance, a passive filter 610 may be replaced with another passive filter 610 configured to increase or decrease the contrast 420 and/or slant 496 of at least one of the left visual stimulus 112 and the right visual stimulus 114.

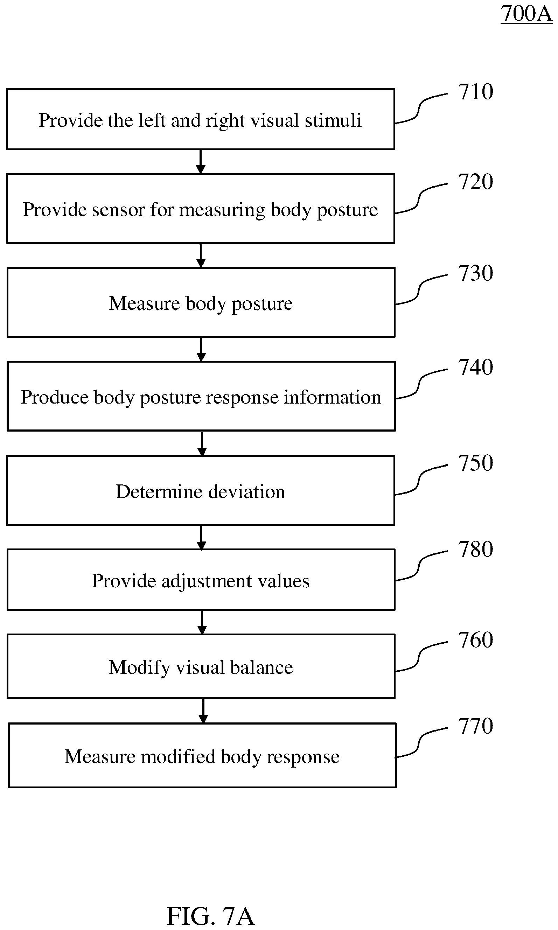

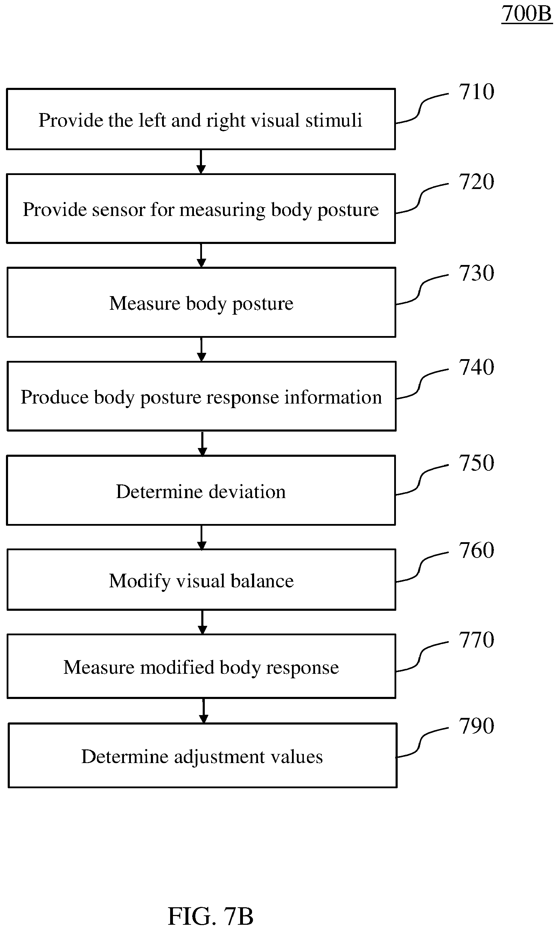

FIGS. 7A and 7B show schematic illustrations of methods 700A, 700B for detecting a body response to a visual stimulus 116, by way of example and in accordance with the various embodiments of the present disclosure. Referring to FIGS. 7A and 7B, method 700A, 700B may include at step 710, providing the left visual stimulus 112 via the left side of the eyewear 110 and the right visual stimulus 114 via the right side of the eyewear 110. For example, the eyewear 110 may be configured to provide the left 112 and right 114 visual stimuli, or may include a filter 610 configured to provide the left 112 and right 114 visual stimuli. At step 720, the method may include providing the sensor 120 for measuring the body posture 140 of the subject's body when the subject is wearing the eyewear 110. For example, the sensor 120 may be attached to a part of, or the entire body of the subject. As a further example, the sensor 120 may be in the external display 620. At step 730, the sensor 120 measures the body posture 140 of the subject, which may be in response to the left 112 and right 114 visual stimuli. Method 700A may include at step 740, producing, using the controlling circuit 130, the body posture response information 150 based on measurements of the body posture 140. For example, the sensor 120 may provide the measurements of the body posture 140 to the controlling circuit 130 via wired or wireless communications, and the controlling circuit 130 may produce the body posture response information 150 based on said measurements and in accordance with the first calculation algorithm. At step 750, the controlling circuit 130 may determine, the deviation 160 of the body posture response information 150 to the reference, which is based on the left visual stimulus 112 and the right visual stimulus 114. Alternatively, the deviation 160 of the body posture response information 150 to the reference may be determined by another microprocessor (e.g. computer, server in a cloud network via wireless communications). According to various embodiments, the deviation 160 may be calculated in accordance with the second calculation algorithm, and may include the subtraction of the body posture response information 150 to the reference.

Referring to method 700A in FIG. 7A and in accordance with one embodiment, step 780 may include providing, using the controlling circuit 130, the set of adjustment values 320 based on the deviation 160 which is determined at step 750 (e.g. based on the second calculation algorithm). The set of adjustment values 320 may be provided to minimize the deviation 160 between the body posture response information 150 and the reference, or to reach the reference. Therefore, at step 760, method 700A may include modifying the visual balance 310 between the left visual stimulus 112 and the right visual stimulus 114, based on the set of adjustment values 320 provided at step 780. For example, modifying the visual balance 310 may include a translation 400, rotation 420, a change in the luminance 440, contrast 460, spatial frequency content 490, slant 496, or a combination thereof, at least one of the left visual stimulus 112 and the right visual stimulus 114. At step 770, the sensor 120 may further measure the body posture 140 of the subject in response to the modified visual balance 310.

Referring to method 700B in FIG. 7B and in accordance with another embodiment, step 790 may include providing, using the controlling circuit 130, the modification of the visual balance 310 between the left visual stimulus 112 and the right visual stimulus 114 to determine the set of adjustment values 320. For example, at step 760, the controlling circuit 130 may be in accordance with the third calculation algorithm, which may include the random modification of the visual balance 310 between the left visual stimulus 112 and the right visual stimulus 114, to determine the set of adjustment values 320. At step 770, the sensor 120 may measure the body posture 140 of the subject in response to the randomly modified visual balance 310. At step 790, the set adjustment values 320 may thus be attained when the body posture 140 of the subject in response to the modification in visual balance 310 reaches that of the reference, or when the body posture 140 of the subject in response to the modification in visual balance 310 is minimized to a pre-determined level (e.g. a level which may be "clinically acceptable", or a level which does not significantly affect the postural balance of the subject).

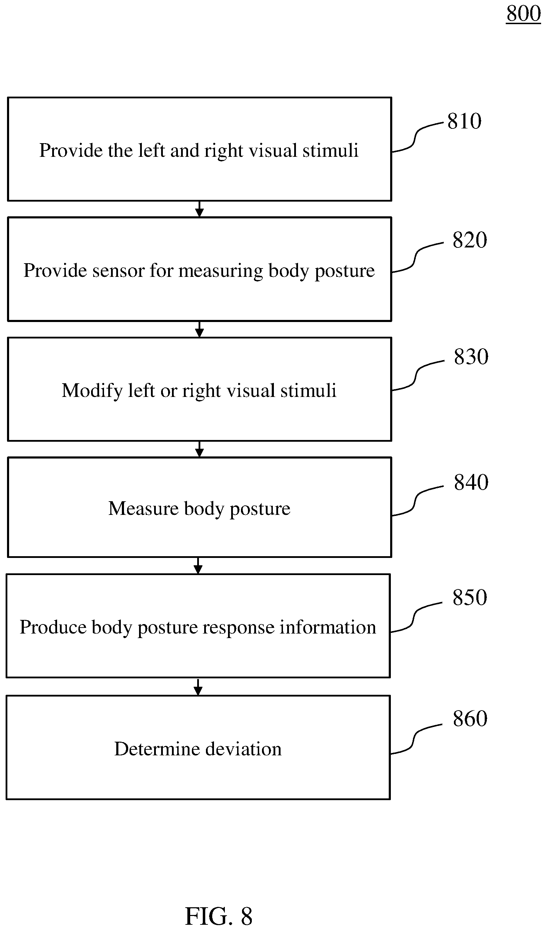

FIG. 8 shows an exemplary schematic illustration of method 800 for detecting a body response to a visual stimulus 116, in accordance with various embodiments. Method 800 includes at step 810, providing the visual stimulus 116 as a left visual stimulus 112 via a left side of an eyewear 110 and a right visual stimulus 114 via a right side of the eyewear 110. For example, the eyewear 110 is configured, or may use a filter 610, to alter a property of the visual stimulus 116 such that the visual stimulus 116 is dichoptically presented. At step 820, a sensor 120 is provided to measure a body posture 140 of a subject's body when the subject is wearing the eyewear 110. Thus, the sensor 120 measures the body posture 140 of the subject's body in response to the dichoptically presented visual stimulus 116. Step 830 includes, modifying, at least one of the left visual stimulus 112 and the right visual stimulus 114. According to various embodiments, the modification in visual balance 310 may include a translation 400, rotation 420, a change in luminance 440, contrast 460, spatial frequency content 490, slant 496, or a combination thereof, at least one of the left visual stimulus 112 and the right visual stimulus 114. For example, the modification in visual balance 310 may produce a determined imbalance or induce significant postural adjustment in the subject's body posture 140, for instance, by modifying the visual stimulus to increase the signal provided to the binocularly weaker eye, to decrease the signal provided to the binocularly dominant eye, or combination thereof. At step 840, method 800 includes measuring the body posture 140 with the sensor 120, which is in response to the modification in visual balance 310. Step 850 includes, producing by a microprocessor operably coupled to the eyewear 110 and the sensor 120, a body posture response information 150 based on the measurements of body posture 140 obtained at step 840. For example, the microprocessor may include or may be, the controlling circuit 130 described above, and the microprocessor may be configured to calculate the body posture response information 150 according to a first calculation algorithm.

Step 860 includes determining the deviation 160 of the body posture response information 150 to a reference, which is based on the left visual stimulus 112 and the right visual stimulus 114. In accordance with one embodiment, determination of the deviation 160 may be performed by the microprocessor which is operably coupled to the eyewear 110 and the sensor 120, for example, the controlling circuit 130. Therefore, the controlling circuit 130 produces the body posture response information 150 and further determines the deviation 160 of the body posture response information 150 to the reference.

In accordance with another embodiment, determining the deviation 160 may be performed by another microprocessor (e.g. external computer, a server in a cloud network). For example, the body posture response information 150 may be communicated via wired or wireless means to another microprocessor to determine the deviation 160.

According to various embodiments, method 800 may be performed by a computing system configured to execute the steps of method 800. For example, a computer program may include instructions to execute the steps of method 800, and may detect the body response of a subject to the visual stimulus 116.

Advantageously, the present disclosure provides a system and a method that allows for the objective evaluation of ocular dominance and its multi-sensory integration during locomotion. Specifically, the system and methods thereof, (i) uses visual stimuli relevant to real world scenarios; and (ii) integrates binocular balance with other sensory processes. Thus, the present disclosure presents systems and methods for objectively evaluating ocular dominance through other sensory processes, for instance, by integrating ocular dominance with other sensory modalities, which are more applicable to real world multi-sensory scenarios.

While the disclosure has been particularly shown and described with reference to specific embodiments, it should be understood by those skilled in the art that various changes in form and detail may be made therein without departing from the spirit and scope of the invention as defined by the appended claims. The scope of the invention is thus indicated by the appended claims and all changes which come within the meaning and range of equivalency of the claims are therefore intended to be embraced.

**Claims**

1.  A system (100) for detecting a body response to a visual stimulus (116), comprising:

    - an eyewear (110) delivering a left visual stimulus (112) via a left side of the eyewear (110) and a right visual stimulus (114) via a right side of the eyewear (110);
    - a sensor (120) configured to measure a body posture (140) of a subject's body when the subject is wearing the eyewear (110); and
    - a controlling circuit (130) operably coupleable to the eyewear (110) and to the sensor (120), so that when operably coupled, the controlling circuit (130):
    - receives measurements of the body posture (140) from the sensor (120);
    - provides a body posture response information (150) based on the measurements of the body posture (140); and
    - determines a deviation (160) of the body posture response information (150) to a reference, wherein the reference is based on the left visual stimulus (112) and the right visual stimulus (114).

2.  The system (300) of claim 1,

wherein the eyewear (110) is configured to modify a visual balance (310) between the left visual stimulus (112) via the left side of the eyewear (110) and the right visual stimulus (114) via the right side of the eyewear (110),

wherein the sensor (120) further measures the body posture (140) of the subject in response to the modification of the visual balance (310) by the eyewear (110).

3. The system (300) of claim 1 or claim 2,
wherein the modification in visual balance (310) comprises a translation (400), a rotation (420), a slant, or a combination thereof, of at least one of the left visual stimulus (112) and the right visual stimulus (114).

4. The system (300) of any one of claims 2 or claim 3,
wherein the modification in visual balance (310) comprises a change in luminance (440), of at least one of the left visual stimulus (112) and the right visual stimulus (114).

5. The system (300) of any one of claims 2 to 4,
wherein the modification in visual balance (310) comprises a change in contrast (460), of at least one of the left visual stimulus (112) and the right visual stimulus (114).

6. The system (300) of any one of claims 2 to 5,
wherein the modification in visual balance (310) comprises a change in spatial frequency content (490), of at least one of the left visual stimulus (112) and the right visual stimulus (114).

7. The system (100, 300) of any one of claims 1 to 6,

wherein measurements of the body posture (140) comprise a set of measurements of a first displacement (250) in a first direction (D1) and a second displacement (260) in a second direction (D2),

wherein the first direction (D1) is medial-lateral, wherein the second direction (D2) is anterior-posterior and perpendicular to the first direction (D1).

8. The system (100, 300) of any one of claims 1 to 7,
wherein the body posture response information (150) is based on:

(i) the first displacement (250);
(ii) the second displacement (260);
(iii) a ratio (290) between the first displacement (250) and the second displacement (260); or
(iv) a combination thereof.

9. The system (300) of any one of claims 2 to 8,

wherein the controlling circuit (130) is further configured to determine a set of adjustment values (320) and to modify the visual balance (310) between the left visual stimulus (112) and the right visual stimulus (114), to minimize the deviation (160) between the body posture response information (150) and the reference, or to reach the reference.

10. The system (600) according to any one of claims 2 to 9,

wherein the eyewear (110) further comprises a filter (610),
wherein the eyewear (110) is coupled to an external display (620) which displays the visual stimulus (116),
wherein the filter (610) is configured to alter a property of the visual stimulus (116) such that the visual stimulus (116) is displayed as the left visual stimulus (112) and the right visual stimulus (114), and is further configured to modify the visual balance (310) between the left visual stimulus (112) and the right visual stimulus (114).

11. A method (700A, 700B) for the detecting body response to the visual stimulus (116) according to any one of claims 1 to 10, comprising:

- providing (710), the left visual stimulus (112) via the left side of the eyewear (110) and the right visual stimulus (114) via the right side of the eyewear (110);
- providing (720), the sensor (120) for measuring the body posture (140) of the subject's body when the subject is wearing the eyewear (110);
- measuring (730), the body posture (140) with the sensor (120);
- producing (740), using the controlling circuit (130), the body posture response information (150) based on the measurements of the body posture (140); and
- determining (750), using the controlling circuit (130), the deviation (160) of the body posture response information (150) to the reference.

12. The method (700A, 700B) of claim 11, further comprising:

- modifying (760), using the controlling circuit (130), the visual balance (310) between the left visual stimulus (112) via the left side of the eyewear (110) and the right visual stimulus (114) via the right side of the eyewear (110),
- measuring (770), using the sensor (120), the body posture (140) of the subject in response to the modified visual balance (310).

13. The method (700A, 700B) of claim 12, further comprising:

(i) providing (780), using the controlling circuit (130), the set of adjustment values (320) based on the deviation (160) of the body posture response information (150) to the reference, or
(ii) providing (790), using the controlling circuit (130), the modification of the visual balance (310) between the left visual stimulus (112) and the right visual stimulus (114) to determine the set of adjustment values (320),
wherein the set of adjustment values (320) is provided to minimize the deviation (160) between the body posture response information (150) and the reference, or to reach the reference.

14. A method (800) for detecting a body response to a visual stimulus (116), comprising:

- providing (810), a left visual stimulus (112) via a left side of an eyewear (110) and a right visual stimulus (114) via a right side of the eyewear (110);
- providing (820), a sensor (120) for measuring a body posture (140) of a subject's body when the subject is wearing the eyewear (110);
- modifying (830), at least one of the left visual stimulus (112) and the right visual stimulus (114);
- measuring (840), the body posture (140) with the sensor (120);
- producing (850), by a microprocessor (130) operably coupled to the eyewear (110) and to the sensor (120), a body posture response information (150) based on the measurements of body posture (140); and
- determining (860), by the microprocessor or by another microprocessor, a deviation (160) of the body posture response information (150) to a reference, wherein the reference is based on the left visual stimulus (112) and the right visual stimulus (114).

15. A computer program comprising instructions to cause a computing system to execute the steps of the method (800) of claim 14.

100

110

112

114

116

140

120

130

150

160

FIG. 1A

170

116

112    114

172

y

x

FIG. 1B

180

116

112    114

182

y

x

FIG. 1C

FIG. 1D

FIG. 2A

FIG. 2B

FIG. 2C

300

FIG. 3

FIG. 4A

FIG. 4B

440

310

450

452

FIG. 4C

460

310

470

472

474

480

482

484

FIG. 4D

490

492

310

494

FIG. 4E

496

310

498

497

499

y

z

x

FIG. 4F

FIG. 5

600

110

610

610

112

114

140

120

116

620

130

150

160

310

320

FIG. 6

700A

```
┌─────────────────────────────────────┐
│   Provide the left and right visual stimuli   │─── 710
└─────────────────────────────────────┘
                    │
                    ▼
┌─────────────────────────────────────┐
│   Provide sensor for measuring body posture   │─── 720
└─────────────────────────────────────┘
                    │
                    ▼
┌─────────────────────────────────────┐
│          Measure body posture          │─── 730
└─────────────────────────────────────┘
                    │
                    ▼
┌─────────────────────────────────────┐
│  Produce body posture response information  │─── 740
└─────────────────────────────────────┘
                    │
                    ▼
┌─────────────────────────────────────┐
│           Determine deviation           │─── 750
└─────────────────────────────────────┘
                    │
                    ▼
┌─────────────────────────────────────┐
│         Provide adjustment values         │─── 780
└─────────────────────────────────────┘
                    │
                    ▼
┌─────────────────────────────────────┐
│          Modify visual balance          │─── 760
└─────────────────────────────────────┘
                    │
                    ▼
┌─────────────────────────────────────┐
│      Measure modified body response      │─── 770
└─────────────────────────────────────┘
```

FIG. 7A

<u>700B</u>

| Provide the left and right visual stimuli | — 710 |
| Provide sensor for measuring body posture | — 720 |
| Measure body posture | — 730 |
| Produce body posture response information | — 740 |
| Determine deviation | — 750 |
| Modify visual balance | — 760 |
| Measure modified body response | — 770 |
| Determine adjustment values | — 790 |

FIG. 7B

800

```
┌─────────────────────────────────────────┐
│                                          │ ─── 810
│    Provide the left and right visual stimuli │
│                                          │
└─────────────────────────────────────────┘
                    │
                    ▼
┌─────────────────────────────────────────┐
│                                          │ ─── 820
│    Provide sensor for measuring body posture │
│                                          │
└─────────────────────────────────────────┘
                    │
                    ▼
┌─────────────────────────────────────────┐
│                                          │ ─── 830
│    Modify left or right visual stimuli   │
│                                          │
└─────────────────────────────────────────┘
                    │
                    ▼
┌─────────────────────────────────────────┐
│                                          │ ─── 840
│         Measure body posture             │
│                                          │
└─────────────────────────────────────────┘
                    │
                    ▼
┌─────────────────────────────────────────┐
│                                          │ ─── 850
│   Produce body posture response information │
│                                          │
└─────────────────────────────────────────┘
                    │
                    ▼
┌─────────────────────────────────────────┐
│                                          │ ─── 860
│         Determine deviation              │
│                                          │
└─────────────────────────────────────────┘
```

FIG. 8

**EP 4 115 795 A1**

## PARTIAL EUROPEAN SEARCH REPORT

under Rule 62a and/or 63 of the European Patent Convention.
This report shall be considered, for the purposes of
subsequent proceedings, as the European search report

Application Number

EP 21 30 5948

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/156965 A1 (GEISINGER DARIO [IL] ET AL) 8 June 2017 (2017-06-08)<br>* figures 1a, 1c, 2, 3 *<br>* paragraph [0025] *<br>* paragraph [0072] – paragraph [0097] *<br>* paragraph [0113] – paragraph [0121] *<br>----- | 1-15 | INV.<br>A61B5/00<br>A61B5/11<br>A61B5/16 |
| X | US 2019/365594 A1 (GEISINGER DARIO [IL] ET AL) 5 December 2019 (2019-12-05)<br>* figures 1a, 1c, 2, 3 *<br>* paragraph [0117] – paragraph [0174] *<br>----- | 1-15 | |
| A | US 9 805 612 B2 (BAER NIKOLAUS [US]; BAER WOLFGANG [US]; NASCENT SYSTEMS INC [US]) 31 October 2017 (2017-10-31)<br>* column 7, line 41 – column 8, line 16 *<br>----- | 4,5 | |
| A | US 2012/127426 A1 (BACKUS BENJAMIN T [US] ET AL) 24 May 2012 (2012-05-24)<br>* figure 1 *<br>* paragraph [0070] *<br>* paragraph [0088] – paragraph [0089] *<br>-----<br>-/-- | 1-15 | TECHNICAL FIELDS SEARCHED (IPC)<br>A61B |

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:
**see sheet C**

| Place of search | Date of completion of the search | Examiner |
|---|---|---|
| The Hague | 13 January 2022 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**page 1 of 2**

31

Europäisches
Patentamt

European
Patent Office

Office européen
des brevets

## PARTIAL EUROPEAN SEARCH REPORT

**Application Number**

**EP 21 30 5948**

| | DOCUMENTS CONSIDERED TO BE RELEVANT | | | CLASSIFICATION OF THE APPLICATION  (IPC) |
|---|---|---|---|---|
| Category | Citation of document with indication, where appropriate, of relevant passages | Relevant to claim | | |
| A | CN 106 511 044 A (CHANGSHA SHUANGQI MEDICAL TECH CO LTD) 22 March 2017 (2017-03-22) * figure 1 * * the whole document * ----- | 1-15 | | |
| A | US 9 662 265 B2 (UNIV OF LOUISVILLE [US]; SALUS UNIV [US]) 30 May 2017 (2017-05-30) * figures 1-4, 6-8, 12-15, 19, 21, 23, 25-26 * ----- | 1-15 | | |

TECHNICAL FIELDS
SEARCHED      (IPC)

EPO FORM 1503 03.82 (P04C10)

page 2 of 2

32

Europäisches
Patentamt
European
Patent Office
Office européen
des brevets

**INCOMPLETE SEARCH**
**SHEET C**

Application Number

**EP 21 30 5948**

Claim(s) completely searchable:
        1-10

Claim(s) searched incompletely:
        11-15

Reason for the limitation of the search:

1        Claims 1, 15, 11, 14 have been drafted as separate independent claims in the same category.
2        Under Article 84 in combination with Rule 43(2) EPC an application may contain more than one independent claim in a particular category only if the subject matter claimed falls within one or more of the exceptional situations set out in paragraphs (a), (b) or (c) of Rule 43(2) EPC. This is not the case in the present application however, for the following reasons:
3        The independent claims do not relate to a plurality of inter-related products (e.g. in the way a plug and a socket can be inter-related) since they do not define co-operating features.
4        Further, the subject-matter of the independent claims does obviously not involve different uses of a product or apparatus, since none of the claims is directed to a use.
5        Further, the independent claims do not provide alternative solutions to a particular problem, where it is not appropriate to cover these alternatives in a single claim, since they relate effectively to the same subject-matter and differ from each other only in respect of the terminology used.
6        Consequently, the claims fail to comply with the requirements set by Article 84 in combination with Rule 43(2) EPC.

## ANNEX TO THE EUROPEAN SEARCH REPORT
## ON EUROPEAN PATENT APPLICATION NO.

EP 21 30 5948

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2022

| Patent document cited in search report | | Publication date | Patent family member(s) | | | Publication date |
|---|---|---|---|---|---|---|
| US 2017156965 | A1 | 08-06-2017 | GB | 2544906 | A | 31-05-2017 |
| | | | US | 2017156965 | A1 | 08-06-2017 |
| | | | WO | 2016001902 | A1 | 07-01-2016 |
| US 2019365594 | A1 | 05-12-2019 | CA | 2953752 | A1 | 06-07-2018 |
| | | | CN | 110520032 | A | 29-11-2019 |
| | | | EP | 3565453 | A1 | 13-11-2019 |
| | | | US | 2019365594 | A1 | 05-12-2019 |
| | | | WO | 2018127851 | A1 | 12-07-2018 |
| US 9805612 | B2 | 31-10-2017 | NONE | | | |
| US 2012127426 | A1 | 24-05-2012 | NONE | | | |
| CN 106511044 | A | 22-03-2017 | NONE | | | |
| US 9662265 | B2 | 30-05-2017 | US | 2016128893 | A1 | 12-05-2016 |
| | | | WO | 2014197489 | A1 | 11-12-2014 |

EPO FORM P0459

For more details about this annex : see Official Journal of the European Patent Office, No. 12/82